# EUROPEAN PATENT APPLICATION

(11) **EP 4 390 396 A1**
(43) Date of publication of application: **26.06.2024**
(21) Application number: 22216411.3
(22) Date of filing: 23.12.2022
(51) Int. Cl.: G01N 33/543, G01N 33/68

(54) **LATERAL FLOW DEVICE FOR THE DETECTION OF ACUTE AORTIC SYNDROME**

(71) Applicant: PeriCard Check GmbH, 28757 Bremen (DE)
(72) Inventor: Alhowaizy, Saman F., 28757 Bremen (DE)
(74) Representative: Peters, Hajo

(57) **Abstract**

The invention relates to a lateral flow device for the detection of at least one protein (AAD-protein) released during an event leading to an acute aortic syndrome/acute aortic dissection (AAS/AAD) in a sample of bodily fluid of a human/patient, the device comprising a strip, a sample pad for receiving the sample of the bodily fluid, a first conjugate pad with a conjugate comprising a protein antibody binding to said AAD-protein and a first detection band comprising a first conjugate-binding protein so that the presence of said AAD-protein in the sample is indicated by a visible color change, wherein the AAD-protein is selected from a group of biomarkers for AAS/AAD including D-dimer smooth muscle actin myosin isozyme CK-MB and nucleic acid component (DISAMIN). In addition, the invention relates in other aspects also to a method for the detection of an AAD-protein in the blood of a patient using the lateral flow device according to the invention, a kit comprising this device as well as a method for determination of the presence of AAD in a patient using an immunoassay.

## Description

### Field of the Invention

The invention relates to a lateral flow device for the detection of at least one protein (AAD-protein) released during an event leading to an acute aortic syndrome/acute aortic dissection (AAS/AAD) in a sample of bodily fluid of a h-uman/patient, the device comprising a strip, a sample pad for receiving the sample of the bodily fluid, a first conjugate pad with a conjugate comprising a protein antibody binding to said AAD-protein and a first detection band comprising a first conjugate-binding protein so that the presence of said AAD-protein in the sample is indicated by a visible color change, wherein the AAD-protein is selected from a group of biomarkers for AAS/AAD including D-dimer smooth muscle actin myosin isozyme CK-MB and nucleic acid component (DISAMIN). In addition, the invention relates in other aspects also to a method for the detection of an AAD-protein in the blood of a patient using the lateral flow device according to the invention, a kit comprising this device as well as a method for determination of the presence of AAD in a patient using an immunoassay.

### Background of the Invention

Acute aortic dissection (AAD) is a life-threatening clinical entity requiring urgent surgical intervention. It is one aspect of acute aortic syndrome (AAS). The peak incidence of aortic dissection is reported to occur in the sixth and seventh decades of life with 2000 new cases per year in North America and 3000 in Europe.^{1,2} The initiating event is an intimal tear due to congenital defects of the aortic wall, such as in Marfan syndrome (MFS), Ehlers-Danlos syndrome (a heterogeneous group connective tissue disorders), idiopathic Erdheim Gsell medial necrosis, bicuspid aortic valve (the most common congenital heart dissection), or even acquired intima damage caused by hypertension, thoracic trauma or other unknown mechanism.³⁻⁵ Subintima or media necrosis results in intimal tear, which eventually allows blood to enter into the aortic wall, thus leading to separation of the aortic wall layers and hence to a rapid propagation of the aortic dissection. The separation of the media and the adventitia layers of the aortic wall may lead to malperfusion of numerous organs with devastating complications.^{2,6} An important clinical fact is that aortic dissections occur in both aneurysmatic expanded calibre as well as in normal calibre of the aorta. The most feared complication of aortic dissection is its outward rupture, which is associated with high mortality.' The surgical treatment depends on the location of the initial tear and on the longitudinal propagation of the dissection. To describe dissections various classifications have been proposed, with the DeBakey and the Stanford classification more often used.^{8,9} When the ascending aorta is affected (Stanford type A), the mortality of untreated patients is about 36%-72% within the first 48 hours and 62%-91 % within the first week.¹⁰⁻¹³ Chest pain and other symptoms of ADD are often confused with those of cardiac infarction, thus complicating the diagnosis and delaying the treatment of AAD. Therefore, a better understanding of the molecular mechanisms underlying AAD can be the first step to supporting the future development of a rapid test for monitoring patients at high risk.

Given that fact it would be a huge benefit to provide a diagnostic antibody test kit that can suitably detect one or more protein/s released during an event leading to an acute aortic syndrome/acute aortic dissection in samples from a human/patient, especially in a timely manner as time is all-important in this indication.

Accordingly, it would be particularly desirable to provide a lateral flow test. Lateral flow (LF) tests are membrane(strip)-based immunoassay tests. Lateral flow devices have a test strip consisting of a membrane, such as porous paper or a sintered polymer, which enables capillary flow of a sample and detection reagents. The membrane(strip) also holds substances that form test and control lines. One end of the membrane(strip) is fitted with a sample pad that contacts another pad used to store the detection conjugate. The other end of the membrane(strip) has a wicking pad that holds fluids and reagents that have migrated via capillary action through the membrane(strip). Lateral flow devices exist for a wide range of targets including the detection of infectious agents, metabolic molecules, antibodies, toxins and drugs for use in human and veterinary applications.

Lateral flow tests are typically modelled on existing immunoassay formats and can be sandwich assays or competitive assays. Many assay variations are possible, but a positive signal is usually achieved by the specific accumulation of a detection complex at the test line.

Lateral flow devices usually need to be specifically developed in an iterative process for the desired application. Each component of the test strip of a lateral flow device requires specific evaluation followed by testing to determine the optimal combination of components. The identification of the most suitable membrane is an important step in the test strip development as this determines the capillary flow properties that need to be compatible with the type of sample to be tested.

A point-of-care lateral flow test that is rapid, reliable and precise would be extremely useful in this regard and the current invention is exactly providing this.

As said above, the initiating event is an intimal tear due to defects of the aortic wall. The dissection of these layers occurs after the aortic rupture. After 5-10 min of the onset of pain, the biological marker (e.g. DISAMIN: D-dimer smooth muscle actin myosin isozyme CK-MB and nucleic acid component; but could also be HMGI-C, aggrecan, smooth muscle myosin heavy chain, or soluble aortic elastin) is released in the blood. The biomarker level remains elevated for 6-10 days since the pain onset, thereby providing a greater one-time window for the detection of aortic wall damage. The high specificity of the biomarker measurements to the aortic wall damage was verified during surgical interventions, which consequently become a biomarker indicative of aortic dissection.

### Summary of the Invention

In one aspect, the present invention provides a lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1 -AAD-protein in the sample is indicated by a visible color change,
wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin. In a preferred embodiment the 1- AAD-protein is aggrecan or is HMGI-C or is DISAMIN (D-dimer smooth muscle actin myosin isozyme CK-MB and nucleic acid component). In a very preferred embodiment, the 1-AAD-protein is HMGI-C and the lateral flow device further comprises at least one second protein antibody binding to a second protein potentially also released during said event leading to an acute aortic dissection (2-AAD-protein), preferably wherein the 2-AAD-protein is selected from a group of biomarkers for AAD including D-dimer smooth muscle actin myosin isozyme CK-MB and nucleic acid component (DISAMIN), and especially from aggrecan, smooth muscle myosin heavy chain, soluble aortic elastin or D-dimer, especially preferably is D-dimer, so that the 1-AAD-protein is HMGC-I and the 2-AAD-protein is D-dimer. It is also preferred if said protein antibody binding to said 1 -AAD-protein binds to the antigen of the amino acid sequence of SEQ ID NO: 1 or a fragment thereof.

In a further embodiment the lateral flow device according to the invention further comprises at least one MI-protein antibody binding to a protein indicative for acute myocardial infarction (MI-protein) together with a separate MI-detection band comprising a further conjugate-binding protein different from the first and second immobilized conjugate-binding protein immobilized within the strip along a band located substantially perpendicular to the direction of flow of the sample along the strip so that when an at least one mobilized MI-protein antibody sample conjugate in the sample contacts the further conjugate-binding protein in the MI-detection band with optionally the presence of said MI-protein in the sample being indicated by a visible color change. Preferably, the MI-protein is selected from the group consisting of creatinine phosphokinase (CK) or its MB isozyme, cardiac myosin light chain, troponin T (TnT) and human heart fatty acids binding protein.

In a further embodiment the lateral flow device according to the invention further comprises a control line located substantially perpendicular to the direction of flow of the sample along the strip.

In another further embodiment the lateral flow device according to any preceding embodiment, wherein the acute aortic dissection (AAD) is an acute aortic syndrome which comprises aortic dissection, including acute type A aortic dissection, and/or one or more of the following aortic dissections/acute aortic syndromes: like Penetrating Aortic Ulcer and Intramural Hematoma, Thoracic Aortic Aneurysm, Chronic Dissection, Indications with Treatment with Branched and Fenestrated Stent-grafts; Different Therapeutic Modalities for Aortic Arch Dissection Combined with Kommerell's Diverticulum; Secondary Interventions After Endovascular Repair of Aortic Dissections; False Aneurysms Complicating Internal Carotid Artery Dissection; Established Indications for the Invasive Treatment of a Thoracic Aortic Aneurysm; Aortic Arch Dissection: A Controversy of Classification; Treatment Algorithms for Patients with (Sub)acute Type B Aortic Dissections; Descending Thoracic Aortic Dissections; Differences in Aortopathy in Patients with a Bicuspid Aortic Valve with or Without Aortic Coarctation; Angina Chest pain; Dyspnea Accompanied with Hypertension Crisis; Sudden Coma or Shock of Unknown Etiology.

Another aspect of the invention is a kit for detecting a protein released during an event leading to an acute aortic dissection (AAD-protein) in a sample of bodily fluid of a mammal, the kit comprising:
a) a lateral flow assay device as described above and below; and
b) an instruction information on how to use the lateral flow assay device as described above and below.

Also included as a further aspect of the invention is a method for the detection of an AAD-protein in a body fluid of a mammal comprising using a lateral flow assay device as described above and below.

Also included as another further aspect of the invention is a method of the detection of a first protein released during an event leading to an acute aortic dissection (1-AAD-protein) in a sample of bodily fluid of a mammal, the method comprising:
a) placing the sample onto a sample pad of a lateral flow assay device comprising:
   (1) a strip formed of a material enabling capillary flow of fluid along a portion of the strip;
   (2) the sample pad located proximal to one end of the strip for receiving the sample of the bodily fluid;
   (3) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   (4) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
   wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin; preferably is HMGI-C, with said antibody binding to the antigen of the amino acid sequence of SEQ ID NO: 1 or a fragment thereof;
b) interrogating the detection region for the presence of label particles to detect whether said AAD protein is present in the sample.

The present invention also includes a method for the immunoassay determination of acute aortic dissection (AAD) in a patient, said method comprising:
a) incubating a body fluid sample of the patient with
   a. at least one antibody to a first protein released during an event leading to an acute aortic dissection (1-AAD-protein) and
   b. a binding partner B for either 1 -AAD-protein or the antibody
      wherein either the antibody or the binding partner B is conjugated to a detection agent,
      to form an immunological complex containing the detection agent;
b) isolating the immunological complex from the remaining sample; and
c) determining the detection agent either in the isolated complex or in the remaining sample as an indicator of the 1-AAD-protein in the sample;

thereby determining the occurrence of AAD in the patient,
wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin; preferably is HMGI-C, with said antibody binding to the antigen of the amino acid sequence of SEQ ID NO: 1 or a fragment thereof.

### Brief Description of the Drawings

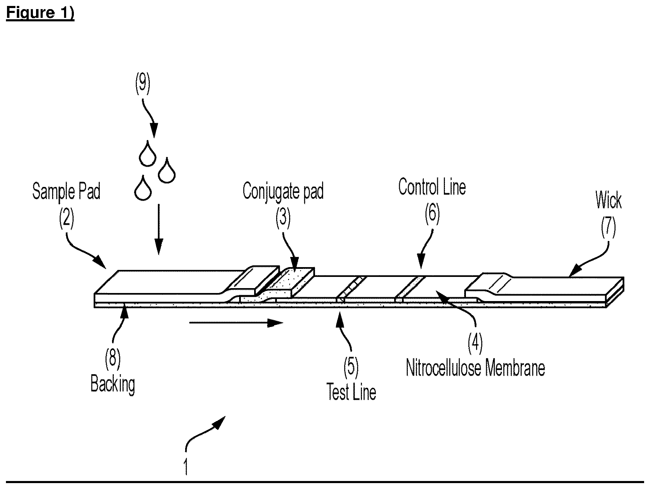

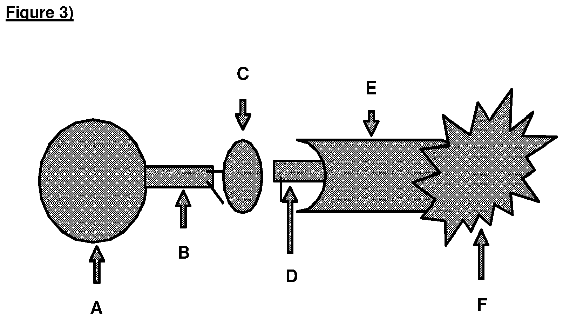

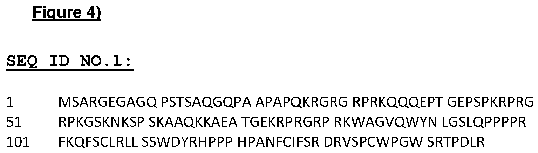

**Figure 1****)** is a schematic representation of a Lateral Flow Device According to the invention. The device (1) has a sample pad (2), a conjugate pad (3) including a mobilizable conjugate comprising an antibody protein binding to said AAD-Protein that has been conjugated to a detection agent, a membrane (4), and an optional wicking pad (7) for receiving and holding fluid that has travelled by capillary flow from the sample pad (2) and through the conjugate pad (3) and the membrane (4). A detection band (5) is shown which comprises an immobilized binding protein. Band (6) is an optional positive control band. The device (1) can further include a backing (8).

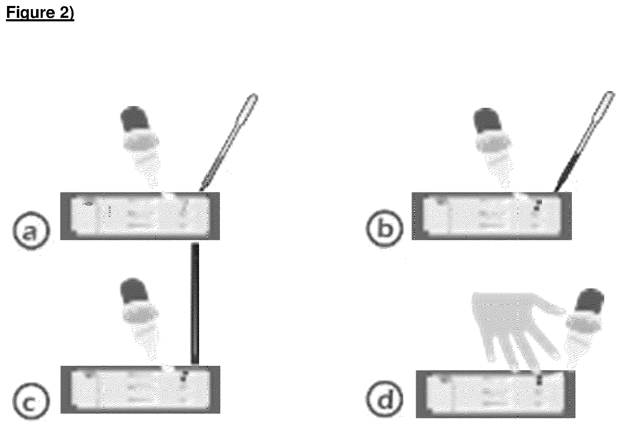

**Figure 2****)** is a schematic presentation of the various ways the sample may be applied to the Lateral Flow Device according to the invention:
   a) For plasma and serum samples.
   b) For venous whole blood.
   c) For whole blood collected from a finger puncture: capillary
   d) For whole blood collected from a finger puncture: hanging drops
**Figure 3****)** presents a schematic example of a complex of the AAD-protein, the conjugate from the conjugate pad and first conjugate-binding protein from the detection band bound to a reporter for the visible color change.
   A: Magnetic Bead
   B: "Capture"-Antibody (a protein antibody binding to said AAD-protein)
   C: The AAD-Protein (the Biomarker)
   D: Biotinylation Detection Antibody (conjugate-binding protein)
   E: Streptavidin
   F: Phycoerythrin-Fluorescent-Reporter
**Figure 4****)** presents SEQ ID NO.: 1, the protein sequence of HMGI-C (High mobility group protein) with 147 amino acids.
**Figure 5****)** presents protein sequences of HMGI-C (High mobility group protein) with different tags used in the example 1 to express, isolate and purify the protein HMGI-C.
**Figure 6****)** presents pictures of the gels showing the purity of the protein HMGI-C derived in Example 1. The conditions were: Reduced SDS-PAGE with Coomassie blue staining; 2µg of sample loaded per lane. MW. Molecular weight marker.

Other objects, aspects, features and advantages of the present invention will become apparent from the following description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### Detailed Description of the invention

### Definitions

Throughout this specification, unless the context requires otherwise, the words "comprise," "comprises," and "comprising" will be understood to imply the inclusion of a stated step or element or group of steps or elements but not the exclusion of any other step or element or group of steps or elements. It should also be understood that a term with the words "comprise," "comprises," and "comprising" can be in a limiting way be replaced to mean the words "consists of", "consists of" and "consisting of", and then be understood to imply the inclusion of a stated step or element or group of steps or elements but the exclusion of any other step or element or group of steps or elements.

The terms "protein", "protein fragment", "polypeptide", and "peptide" may be used interchangeably herein to refer to a polymer of amino acid residues and to variants and synthetic and naturally occurring analogues of the same. Thus, these terms apply to amino acid polymers in which one or more amino acid residues are synthetic non-naturally occurring amino acids, such as a chemical analogue of a corresponding naturally occurring amino acid, as well as to naturally- occurring amino acid polymers and naturally occurring chemical derivatives thereof.

The term "antibody", also referred to as "immunoglobulin", is a Y-shaped protein of the immune system that specifically identifies foreign objects or antigens, such as the components of bacteria, yeasts, parasites, and viruses. Each tip of the 'Y' of an antibody contains an antigen-binding site that is specific for a particular epitope on an antigen, allowing these two structures to bind together with precision. An "antibody" typically comprises all or a portion of an Fc region, to facilitate detection by an Fe-binding protein, and may also comprise one or more antigen-binding sites, to facilitate detection by an antibody-specific binding agent, such as an antigen or antigenic peptide. The antibodies can be, e.g., of IgG, IgE, IgD, IgM, or IgA type.

The term "antigen" means a molecule having distinct surface features or epitopes capable of stimulating a specific immune response. Antibodies (immunoglobulins) are produced by the immune system in response to exposure to antigens. Antigens maybe proteins, carbohydrates or lipids, although only protein antigens are classified as immunogens because carbohydrates and lipids cannot elicit an immune response on their own.

An "antigen-binding site," or "binding portion" of an antibody, refers to the part of the immunoglobulin molecule that participates in antigen binding. The antigen-binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs". In an antibody molecule, the three hypervariable regions of a light chain and the three hypervariable regions of a heavy chain are disposed relative to each other in three dimensional space to form an antigen-binding surface. The antigen-binding surface is complementary to the three dimensional surface of a bound antigen, and the three hypervariable regions of each of the heavy and light chains are referred to as "complementarity-determining regions," or "CDRs."

The term "Fc-binding protein" refers to a protein or polypeptide capable of binding to the fragment crystallizable region (Fc region) of an antibody. The Fc region is the tail region of an antibody that interacts with cell surface Fc receptors and certain proteins of the complement system. In IgG, IgA and IgD antibody isotypes, the Fc region is composed of two identical protein fragments, derived from the second and third constant domains of the antibody's two heavy chains. IgM and IgE Fc regions contain three heavy chain constant domains (CH domains 2-4) in each polypeptide chain. The Fc regions of IgG antibodies bears a highly conserved N-glycosylation site. The N-glycans attached to this site are predominantly core-fucosylated diantennary structures of the complex type. In addition, small amounts of these N-glycans also bear bisecting GlcNAc and. alpha-2,6 linked sialic acid residues. The Fab region of an antibody contains variable sections that define the target-specificity of the antibody, and in contrast, the Fc region of all antibodies in a class are the same for each species; they are constant rather than variable.

The term "nanoparticles" means uniform particles having a size of 1-200 nm.

The term "nanoshells" means nanoparticles that consist of a core and a metallic shell (usually gold).

In the context of this invention the term "acute aortic dissection (AAD)" is to be understood as a clinical entity being part of the "AAS", initiated by an intimal tear due to congenital defects of the aortic wall, such as in Marfan syndrome (MFS), Ehlers-Danlos syndrome (a heterogeneous group connective tissue disorders), idiopathic Erdheim Gsell medial necrosis, bicuspid aortic valve (the most common congenital heart dissection), or even acquired intima damage caused by hypertension, thoracic trauma or other unknown mechanism.³⁻⁵ One complication of aortic dissection is its outward rupture, which is surgically treated. With the Stanford type A the mortality of untreated patients is about 36%-72% within the first 48 hours and 62%-91 % within the first week.¹⁰⁻¹³.

In the context of this invention the term "acute aortic syndrome (AAS)" is to be understood as a broader term including the AAD. The AAS (as well as the AAD) can include the following syndroms: Penetrating Aortic Ulcer and Intramural Hematoma, Thoracic Aortic Aneurysm, Chronic Dissection-Indications with Treatment with Branched and Fenestrated Stent-grafts, Different Therapeutic Modalities for Aortic Arch Dissection Combined with Kommerell's Diverticulum, Secondary Interventions After Endovascular Repair of Aortic Dissections, False Aneurysms Complicating Internal Carotid Artery Dissection, Established Indications for the Invasive Treatment of a Thoracic Aortic Aneurysm, Aortic Arch Dissection: A Controversy of Classification, Treatment Algorithms for Patients with (Sub)acute Type B Aortic Dissections, Descending Thoracic Aortic Dissections, Differences in Aortopathy in Patients with a Bicuspid Aortic Valve with or Without Aortic Coarctation, Angina Chest pain, Dyspnea Accompanied by Hypertension Crisis, Sudden Coma or Shock of Unknown Etiology.

In the context of this invention the term "1-AAD-protein" is to be understood as one or more from a group of proteins of which one or more are released into the blood after the initiating event of the acute aortic syndrome/acute aortic dissection (AAS/AAD). This initiating event is an intimal tear due to defects of the aortic wall. The dissection of these layers occurs after the aortic rupture. After 5-10 min of the onset of pain, one or more of these biological markers, the "1-AAD-protein/s" is/are released in the blood. The biomarker ("1-AAD-protein") level remains elevated for 6-10 days since the pain onset, thereby providing a great one-time window for the detection of aortic wall damage. The high specificity of the biomarker measurements to the aortic wall damage was verified during surgical interventions, which consequently become a biomarker indicative of aortic dissection. The "1-AAD-proteins" can be e.g. DISAMIN: D-dimer smooth muscle actin myosin isozyme CK-MB and nucleic acid component; but it could also be HMGI-C, aggrecan, smooth muscle myosin heavy chain, or soluble aortic elastin. Preferably, it is HMGI-C.

In the context of this invention the term "2-AAD-protein" is to be understood as one or more from a group of proteins of which one or more are released into the blood after the initiating event of the acute aortic syndrome/acute aortic dissection (AAS/AAD). This initiating event is an intimal tear due to defects of the aortic wall. The dissection of these layers occurs after the aortic rupture. After 5-10 min of the onset of pain, one or more of these biological markers, the "2-AAD-protein/s" is/are released in the blood. The biomarker ("2-AAD-protein") level remains elevated for 6-10 days since the pain onset, thereby providing a great one-time window for the detection of aortic wall damage. The high specificity of the biomarker measurements to the aortic wall damage was verified during surgical interventions, which consequently become a biomarker indicative of aortic dissection. The "2-AAD-proteins" can be e.g. DISAMIN: D-dimer smooth muscle actin myosin isozyme CK-MB and nucleic acid component; but it could also be HMGI-C, aggrecan, smooth muscle myosin heavy chain, or soluble aortic elastin.

In the context of this invention the term "X-AAD-protein" is to be understood as one or more from a group of proteins of which one or more are released into the blood after the initiating event of the acute aortic syndrome/acute aortic dissection (AAS/AAD). This initiating event is an intimal tear due to defects of the aortic wall. The dissection of these layers occurs after the aortic rupture. After 5-10 min of the onset of pain, one or more of these biological markers, the "X-AAD-protein/s" is/are released in the blood. The biomarker ("X-AAD-protein") level remains elevated for 6-10 days since the pain onset, thereby providing a great one-time window for the detection of aortic wall damage. The high specificity of the biomarker measurements to the aortic wall damage was verified during surgical interventions, which consequently become a biomarker indicative of aortic dissection. The "X-AAD-proteins" can be e.g. DISAMIN: D-dimer smooth muscle actin myosin isozyme CK-MB and nucleic acid component; but it could also be HMGI-C, aggrecan, smooth muscle myosin heavy chain, or soluble aortic elastin.

In the context of this invention the term "biomarker" is to be understood as any compound of any biological origin whose detection signals the occurrence of a event in an organism. In the context of this invention "biomarker" is especially understood as one or more proteins, especially enzymes, whose presence in a bio-sample for acute aortic dissection (AAD)more especially the above-described "1-AAD-protein/s", "2-AAD-protein/s" or "X-AAD-protein/s".

In the context of this invention the term "sample pad" is to be understood as a stretch on (or a part of) the strip onto which a fluid sample or analyte, preferably a sample of body fluids, especially of blood, e.g. of blood from a human/ a patient is applied. This sample pad can e.g. comprise or consist of polymers or paper or cotton, like sintered polymer, cotton, porous paper or a microstructured polymer or nitrocellulose. The sample pad should be constructed so that the bodily fluid can be - e.g. by way of an opening in the lateral flow device - applied to it. The sample pad can also act as a sponge and is often able to hold excess fluid.

In the context of this invention the term "bodily fluid" is to be understood as any fluid inside a body, especially that of a mammal, like a human or a patient. A body fluid or bodily fluid can be blood, urine, semen, sputum, saliva etc., most preferably is blood like whole blood, blood serum etc.

In the context of this invention the term "strip" is to be understood as a material or combination of materials enabling capillary flow of fluid along - at least - a portion of the strip. The strip may have - and preferably has - the capacity to spontaneously transport the fluid, like bodily fluid, like blood. The strip can comprise or consist of polymers or paper, like sintered polymer, a microstructured polymer, nitrocellulose, porous paper, especially porous paper like nitrocellulose.

In the context of this invention the term "conjugate" is to be understood as a protein antibody binding to a biomarker (see above) that has been conjugated to a detection agent. In a preferred embodiment said protein antibody is an antibody that binds to a 1-AAD-protein, 2-AAD-protein or X-AAD-protein (see above) and which is conjugated to a detection agent (a Tag) like gold, colored latex etc. Often, the antibody is freeze-dried and/or is a monoclonal antibody. Preferably the antibody in the conjugate is an antibody binding to HMGI-C, more preferably is a monoclonal antibody binding to HMGI-C, most preferably is a freeze-dried monoclonal antibody binding to HMGI-C. Preferably in the conjugate this antibody is conjugated to a gold-particle or a colored latex-particle, more preferably a gold particle. Most preferably the conjugate is a monoclonal antibody binding to HMGI-C conjugated with a gold-particle.

In the context of this invention the term "conjugate pad" is to be understood as a stretch on (or a part of) the strip into which the fluid sample or analyte, previously applied to the sample pad, is transported via capillary flow, e.g. in the strip. It does contain (comprise) a conjugate (see above). This conjugate pad can e.g. consist of polymers or paper, like sintered polymer, porous paper or a microstructured polymer or nitrocellulose. When the sample or analyte flows under capillary action through the strip from the sample pad to the conjugate pad, it mobilizes the conjugate contained (comprised) in the conjugate pad.

In the context of this invention the term "mobilize" is to be understood as being displaced from its current position, e.g. inside the conjugate pad. Preferably, this is applied to the movement of the conjugate (see above) contained inside the conjugate pad further along the strip along with the sample/fluid, e.g. by capillary forces, e.g. towards/to the "detection band". Most preferably, this is applied to the movement of the conjugate (see above) contained inside the conjugate pad which is bound through its protein antibody to the biomarker (1-AAD-protein, 2-AAD-protein, or X-AAD-protein) from the sample/analyte further along the strip along with the sample/fluid.

In the context of this invention "the 1-AAD-protein antibody conjugate" (or the "2-AAD-protein antibody conjugate") is the conjugate (see above) of the "protein antibody binding to" the "biomarker", 1-AAD-protein (or 2-AAD-protein), with a "detection agent". This also covers the conjugate (see above) of the "protein antibody binding to" the "biomarker", 1-AAD-protein (or 2-AAD-protein), with a "detection agent" being bound through its protein antibody to the biomarker 1-AAD-protein (or 2-AAD-protein).

In the context of this invention "the mobilized 1-AAD-protein antibody conjugate" (or the mobilized 2-AAD-protein antibody conjugate) is the conjugate (see above) of the "protein antibody binding to" the "biomarker", 1-AAD-protein (or 2-AAD-protein), with a "detection agent", being mobilized e.g. from the conjugate pad and moved along with the sample. This also covers the conjugate (see above) of the "protein antibody binding to" the "biomarker", 1-AAD-protein (or 2-AAD-protein), with a "detection agent" being bound through its protein antibody to the biomarker 1-AAD-protein (or 2-AAD-protein), being mobilized e.g. from the conjugate pad and moved along with the sample.

In the context of this invention the term "protein antibody" is to be understood as an antibody being a protein that binds to a biomarker (see above). In a preferred embodiment said protein antibody is an antibody that binds to a 1-AAD-protein, 2-AAD-protein or X-AAD-protein (see above). Often, the antibody is freeze-dried and/or is a monoclonal antibody. Preferably the protein antibody is an antibody binding to HMGI-C, more preferably is a monoclonal antibody binding to HMGI-C. It can also be preferred that the protein antibody is an antibody binding to D-Dimer, more preferably is a monoclonal antibody binding to D-Dimer. It can also be preferred that the protein antibody is an antibody binding to the antigen of the amino acid sequence of SEQ ID NO: 1 or a fragment thereof SEQ ID No. 1, more preferably is a monoclonal antibody binding to the antigen of the amino acid sequence of SEQ ID NO: 1 or a fragment thereof.

In the context of this invention the term "protein antibody binding to" is to be understood as a protein antibody wherein it is clarified to which antigen it binds. Usually the "protein antibody binding to" binds to a biomarker (see above) and in a preferred embodiment said protein antibody binds to a 1-AAD-protein, 2-AAD-protein or X-AAD-protein (see above) as its antigen. Preferably it is a protein antibody binding to HMGI-C, more preferably is a monoclonal antibody binding to HMGI-C.

In the context of this invention the term "detection agent" is to be understood as a tag like gold, colored latex, or gold and colored latex-particles or magnetic beads. Preferably the detection agent is understood as part of the conjugate described above, being preferably a gold or latex particle being conjugated to the protein antibody (see above).

In the context of this invention the term "detection band" is to be understood as a stretch on the strip into which the fluid sample or analyte together with the mobilized conjugate (which comprises the "protein antibody"), contained in the conjugate pad, is transported via capillary flow, e.g. in the strip. The detection band is located substantially perpendicular to the direction of flow of the sample along the strip. The detection band does comprise (contain) a (first or second) conjugate-binding protein (see below). The (first or second) conjugate-binding protein is immobilized within the strip (the detection band), preferably along a band (the detection band) located substantially perpendicular to the direction of flow of the sample along the strip. Thus, when the flow of the sample together with the mobilized (1- or 2-)AAD-protein antibody conjugate in the sample reaches the detection band, it contacts (or - at least some AAD-protein antibody conjugate - binds to) the first conjugate-binding protein comprised (contained) within the detection band. This leads to that the presence of said (1- or 2-)AAD-protein in the sample is indicated by a visible color change. This detection band can e.g. consist of polymers or paper, like sintered polymer, porous paper or a microstructured polymer or nitrocellulose.

In the context of this invention the term "immobilized" is to be understood as first of all meaning that it is comprised/contained within the detection band and within the strip. Preferably, it means that it is not mobilized when fluid contacts the immobilized entity, even if continues to flow by capillary flow. Thus, it means that it (e.g. the immobilized (first or second) conjugate-binding protein) is not mobilized by the fluid sample if it (together with the mobilized (1- or 2-)AAD-protein antibody conjugate in the sample) reaches (and contacts) the "immobilized" (first or second) conjugate-binding protein in the detection band.

In the context of this invention the term "contact" or "contacts" is to be understood as one entity getting in contact with another entity. It also includes "binding" or "binds". Thus, "contacts" also includes if a "conjugate-binding-protein" binds to a (mobilized) "AAD-protein antibody conjugate" like a "mobilized 1-AAD-protein antibody conjugate".

In the context of this invention the term "conjugate binding site" is to be understood as covering two different embodiments: a) the "conjugate-binding protein" (as described directly below) or b) a construct in which a surface-structure (site) of the biomarker, the 1-AAD-protein, 2-AAD-Protein or X-AAD-protein is presented to the fluid, e.g. of the sample. In this, the conjugate binding site is immobilized within the strip, usually at a detection band, usually the first detection band, but it could also be immobilized in another (2^{nd}) detection band. In that, in a sample containing the mobilized 1-AAD-protein antibody conjugate (the mobilized 2-AAD-protein antibody conjugate), the mobilized 1-AAD-protein antibody conjugate (the mobilized 2-AAD-protein antibody conjugate) could bind to the conjugate binding site if the "protein antibody" is not already bound to the biomarker/AAD-protein.

In the context of this invention the term "conjugate-binding protein" is to be understood as a protein that is binding to a conjugate comprising an antibody. Preferably, the conjugate-binding protein is binding to the "protein antibody" (described above). The "conjugate-binding protein" binds a) either directly to the "protein antibody" (described above) being part of the "conjugate" (descirbed above) or b) indirectly to the "protein antibody" (described above) by binding to the biomarker as an antigen (1-AAD-protein, 2-AAD-protein, or X-AAD-protein) which is bound via the "protein antibody" of the "conjugate" (described above). Most importantly, though, the "conjugate-binding protein" is - highly preferably - only binding to the protein antibody of the conjugate - whether directly or indirectly - if the protein antibody of the conjugate is - preferably already - bound to the biomarker/AAD-protein. This would be the case in a preferred embodiment of the invention in which the LFA is executed as a Sandwich assay. It is to be understood that the term "contact" also includes "binding" or "binds" in the context of "conjugate-binding protein". The "conjugate-binding protein" is immobilized within the strip, usually at a detection band, usually the first detection band, but it could also be immobilized in another (2^{nd}) detection band. In a preferred embodiment, the "conjugate-binding protein" is directly binding to a biomarker as antigen, which is also bound to the protein antibody of the conjugate; the conjugate being mobilized from the conjugate pad by the fluid from the sample with the protein antibody of the conjugate bound to the biomarker. In a very preferred embodiment, the "conjugate-binding protein" is directly binding to HMGI-C, which is also bound to the protein antibody of the conjugate; the conjugate being mobilized from the conjugate pad by the fluid from the sample with the protein antibody of the conjugate bound to HMGI-C. As an alternative the "conjugate-binding protein" is an FC-binding protein, preferably wherein the FC-binding protein is Protein G, Protein A or a Protein A/G fusion protein. If the "conjugate-binding protein" binds to the same biomarker as the "protein antibody" (in the "conjugate"), the "conjugate-binding protein" can bind to the same epitope on the same biomarker as the "protein antibody" (in the "conjugate") or the "conjugate-binding protein" can bind to a different epitope on the same biomarker than the "protein antibody" (in the "conjugate").

In the context of this invention the term "further conjugate-binding protein" is to be understood as a protein that is binding to a mobilized MI-protein antibody sample conjugate comprising an antibody to an MI-protein (see below). The further conjugate-binding protein" is different from the first and second immobilized conjugate-binding protein. The "further conjugate-binding protein" binds a) either directly to the "MI-protein antibody" (described below) being part of a conjugate or b) indirectly to the "MI-protein antibody" (described below) by binding to the MI-protein, 2-AAD-protein, or X-AAD-protein) which is bound via the "MI-protein antibody" being conjugated. The "further conjugate-binding protein" is only binding to the MI-protein antibody being conjugated - whether directly or indirectly - if the MI-protein antibody being conjugated is bound to the MI-protein.

In the context of this invention the term "the presence or absence of color" is to be understood as either a) a "visible color change" of the detection band or b) no change of color in the detection band when the mobilized (1-; or 2-)AAD-protein antibody conjugate in the sample contacts the (first or second) conjugate-binding protein in the (first or second) detection band.

In the context of this invention the term "visible color change" is to be understood as a change of color that is - usually - detectable by the naked eye in the detection band when the mobilized (1-; or 2-)AAD-protein antibody conjugate in the sample contacts the (first or second) conjugate-binding protein in the (first or second) detection band. An example is the color change from white to a red color if the "conjugate" is comprising gold particles or from white to a blue color if the "conjugate" is comprising blue Latex particles.

In the context of this invention the term "DISAMIN" is an acronym covering D-dimer smooth muscle actin myosin isozyme CK-MB and nucleic acid component. Accordingly, the group of biomarkers for AAD, denominated by "DISAMIN" includes D-dimer smooth muscle actin myosin isozyme CK-MB and nucleic acid component, and thus especially "D-dimer", "smooth muscle actin" "myosin heavy chain" and also "isozyme CK-MB".

In the context of this invention the term "D-dimer" is to be understood as a specifc degradation product of cross-linked fibrin that is normally produced by plasmin hydrolysis. A cardiac "D-dimer" assay is used for detection of pulmonary embolism and deep vein thrombosis.

In the context of this invention the term "smooth muscle actin" is to be understood as an actin protein that is a globular multi-functional protein that form microfilaments and is involved in the contractile apparatus of smooth muscle.

In the context of this invention the term "smooth muscle myosin heavy chain" is to be understood as the heavy chain of myosin from smooth muscle cells,

In the context of this invention the term "cardiac myosin light chain" is to be understood as the light chain of myosin from heart muscle cells.

In the context of this invention the term "isozyme CK-MB" is to be understood as the creatinekinase creatine kinase myocardial band being the bound combination of two variants (isoenzymes CKM and CKB) of the enzyme phosphocreatine kinase.

In the context of this invention the term "creatinine phosphokinase (CK) or its MB isozyme" (also CPK) is to be understood as synonymous to creatine kinase (CK) wherein MB signifies "myocardial band".

In the context of this invention the term "troponin T (TnT)" is to be understood as a part of troponin (or the troponin complex). It is integral to muscle contraction in skeletal muscle and cardiac muscle, but not smooth muscle.

In the context of this invention the term "HMGI-C" (also HNGA2) is to be understood as a protein that belongs to the non-histone chromosomal high-mobility group (HMG) protein family. HMG proteins function as architectural factors and are essential components of the enhanceosome. It may act as a transcriptional regulating factor and its expression in adult tissues is commonly associated with both malignant and benign tumor formation.

In the context of this invention the term "Aggrecan" is to be understood as a proteoglycan able to form large aggregates in the cartilage tissue. It is am member of the chondroitin sulfate proteoglycan family and is an integral part of the extracellular matrix in cartilagenous tissue.

In the context of this invention the term "soluble aortic elastin" is to be understood as and elastin that soluble and present at the aorta. Elastin is a key component of the extracellular matrix. It is highly elastic and present in connective tissue allowing many tissues in the body to resume their shape after stretching or contracting. Elastin is used in places where mechanical energy is required to be stored.

In the context of this invention the term "elastin degradation product" is to be understood as the degradation product of elastin.

In the context of this invention the term "human heart fatty acids binding protein" (hFABP) also known as mammary-derived growth inhibitor is a small cytoplasmic protein released from cardiac myocytes following an ischemic episode. It is involved in active fatty acid metabolism where it transports fatty acids from the cell membrane to mitochondria for oxidation.

In the context of this invention the term "acute myocardial infarction" is to be understood as a medical complication that occurs when blood flow decreases or stops to the coronary artery of the heart, causing damage to the heart muscle. It is also commonly known as a heart attack.

In the context of this invention the term "MI-protein" is to be understood as a protein indicative for acute myocardial infarction. Examples include creatinine phosphokinase (CK) or its MB isozyme, cardiac myosin light chain, troponin T (TnT) and human heart fatty acids binding protein (MI-protein).

In the context of this invention the term "MI-detection band" is to be understood as a stretch on the strip into which the fluid sample or analyte together with the mobilized Ml-protein antibody (sample) conjugate is transported via capillary flow. The MI-detection band is located substantially perpendicular to the direction of flow of the sample along the strip and is usually distanced from the (first r second) detection band. The Ml-detection band does comprise (contain) a further conjugate-binding protein (see below). The further conjugate-binding protein is immobilized within the strip (the MI-detection band), preferably along a band (the MI-detection band) located substantially perpendicular to the direction of flow of the sample along the strip. Thus, when the flow of the sample together with the mobilized MI-protein antibody being conjugated in the sample reaches the MI-detection band, it contacts (or - at least some MI-protein antibody conjugate - binds to) the further conjugate-binding protein comprised (contained) within the MI-detection band. This leads to that the presence of said MI-protein in the sample is indicated by a visible color change. This detection band can e.g. consist of polymers or paper, like sintered polymer, porous paper or a microstructured polymer or nitrocellulose.

In the context of this invention the term "wicking pad" is to be understood as a stretch on (or a part of) the strip into which the fluid sample or analyte is transported via capillary flow, e.g. in the strip. It is at the end of the strip (membrane) opposite the sample pad. It holds the fluids (sample or analyte) and reagents that have migrated via capillary action through the membrane (strip) and acts more or less as a waste container. This wicking pad can e.g. consist of polymers or paper, like sintered polymer, porous paper or a microstructured polymer or nitrocellulose.

In the context of this invention the term "Protein G, Protein A or a Protein A/G fusion protein" is to be understood as a group of proteins that bind to (especially human) IgG, polyclonal or monoclonal antibodies as well as to IgA, IgE, IgM and IgD. Protein A/G is a recombinant fusion protein that combines IgG binding domains of both Protein A and Protein G.

### Lateral Flow Device and Kits

With reference to the figures, Figure 1 shows an example of a Lateral Flow Device (1) according to the invention. The device (1) has a sample pad (2), a conjugate pad (3) including a mobilizable conjugate comprising an antibody protein binding to said AAD-Protein that has been conjugated to a detection agent, a membrane (4), and an optional wicking pad (7) for receiving and holding fluid that has travelled by capillary flow from the sample pad (2) and through the conjugate pad (3) and the membrane (4). A detection band (5) is shown which comprises an immobilized binding protein. Band (6) is an optional positive control band. The device (1) can further include a backing (8).

In one aspect, the present invention provides a lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin.

In a preferred embodiment A of the lateral flow device according to the invention the 1- AAD-protein is aggrecan.

In another, alternative preferred embodiment A of the lateral flow device according to the invention the 1-AAD-protein is HMGI-C.

In a preferred embodiment A of these the lateral flow device according to the invention said protein antibody binds to the antigen of the amino acid sequence of SEQ ID NO: 1 or a fragment thereof.

In a preferred embodiment B of the lateral flow device according to the invention the 1-AAD-protein is HMGI-C and the lateral flow device further comprises a second protein antibody binding to a second protein potentially also released during said event leading to an acute aortic dissection (2-AAD-protein), preferably wherein said protein antibody binding to said 1-AAD-protein binds to the antigen of the amino acid sequence of SEQ ID NO: 1 or a fragment thereof. Preferably, the 2-AAD-protein is selected from a group of biomarkers for AAD including D-dimer smooth muscle actin myosin isozyme CK-MB and nucleic acid component (DISAMIN), and especially from aggrecan, smooth muscle myosin heavy chain, soluble aortic elastin or D-dimer. More prefeably, the 2-AAD-protein is is D-Dimer.

Most preferably in this embodiment B, the 1-AAD-protein is HMGI-C and the 2-AAD-protein is D-Dimer.

It is preferred if in the embodiment B of the preceding paragraph the lateral flow device according to the invention said second protein antibody to the 2-AAD-protein is conjugated to a second detection agent different from the one conjugated to said antibody to the 1-AAD-protein and said conjugate with the second protein antibody to the 2-AAD-protein is either located in said first conjugate pad or in a further conjugate pad; or
said second protein antibody to the 2-AAD-protein is conjugated to the same detection agent as the one conjugated to said antibody to the 1-AAD-protein and said conjugate with the second protein antibody to the 2-AAD-protein is either located in said first conjugate pad or in a further conjugate pad, preferably wherein the first conjugate-binding protein in the first detection band will, when the second mobilized 2-AAD-protein antibody conjugate in the sample contacts the first conjugate-binding protein in the first detection band, the presence of the 2-AAD-protein in the sample is indicated by a visible color change.

It is preferred if in these embodiments B of the preceding paragraph the lateral flow device according to the invention comprises a second detection band comprising a second conjugate-binding protein different from the first immobilized within the strip along a band located distanced from the first detection band substantially perpendicular to the direction of flow of the sample along the strip so that when the mobilized 2-AAD-protein antibody conjugate in the sample contacts the second conjugate-binding protein in the second detection band, the presence of the 2-AAD-protein in the sample is indicated by a visible color change.

In a preferred embodiment C of the lateral flow device according to the invention the lateral flow device further comprises one or more further protein antibody/ies binding to one or more further protein/s potentially also released during said event leading to an acute aortic dissection (X-AAD-protein/s), preferably wherein the 1-AAD-protein and the X-AAD-protein/s is/are selected from a group of biomarkers for AAD including D-dimer smooth muscle actin myosin isozyme CK-MB and nucleic acid component (DISAMIN), and especially from aggrecan, smooth muscle myosin heavy chain, soluble aortic elastin or D-dimer.

In a preferred embodiment D of the lateral flow device according to the invention the 1-AAD-protein is HMGI-C and the lateral flow device further comprises one or more further protein antibody/ies binding to one or more further protein/s potentially also released during said event leading to an acute aortic dissection (X-AAD-protein/s) preferably wherein said protein antibody binds to the antigen of the amino acid sequence of SEQ ID NO: 1 or a fragment thereof, preferably wherein the X-AAD-protein/s is/are selected from a group of biomarkers for AAD including D-dimer smooth muscle actin myosin isozyme CK-MB and nucleic acid component (DISAMIN), and especially from aggrecan, smooth muscle myosin heavy chain, soluble aortic elastin or D-dimer.

In a preferred embodiment C or D of the lateral flow device according to the invention said one or more further protein antibody/ies to the X-AAD-protein/s is/are conjugated to the same detection agent as the one conjugated to said antibody to the 1-AAD-protein and said conjugate with the one or more further protein antibody/ies to the X-AAD-protein/s is/are located in said first conjugate pad and the first conjugate-binding protein in the first detection band will when the one or more further mobilized X-AAD-protein/s antibody/ies conjugate/s in the sample contact/s the first conjugate-binding protein in the first detection band, the presence of the one or more X-AAD-protein/s in the sample is/are indicated by a visible color change.

In another preferred embodiment of the lateral flow device according to the invention, the lateral flow device further comprises at least one MI-protein antibody binding to a protein indicative for acute myocardial infarction (MI-protein) together with a separate Ml-detection band comprising a further conjugate-binding protein different from the first and second immobilized conjugate-binding protein immobilized within the strip along a band located substantially perpendicular to the direction of flow of the sample along the strip so that when an at least one mobilized MI-protein antibody sample conjugate in the sample contacts the further conjugate-binding protein in the MI-detection band the presence of said MI-protein in the sample is indicated by a visible color change, preferably wherein the MI-protein is selected from the group consisting of creatinine phosphokinase (CK) or its MB isozyme, cardiac myosin light chain, troponin T (TnT) and human heart fatty acids binding protein.

In another preferred embodiment of the lateral flow device according to the invention the acute aortic dissection (AAD) is an acute aortic syndrome which comprises aortic dissection, including acute type A aortic dissection, and/or one or more of the following aortic dissections/acute aortic syndroms:
- Penetrating Aortic Ulcer and Intramural Hematoma
- Thoracic Aortic Aneurysm
- Chronic Dissection-Indications with Treatment with Branched and Fenestrated Stent-grafts
- Different Therapeutic Modalities for Aortic Arch Dissection Combined with Kommerell's Diverticulum
- Secondary Interventions After Endovascular Repair of Aortic Dissections
- False Aneurysms Complicating Internal Carotid Artery Dissection
- Established Indications for the Invasive Treatment of a Thoracic Aortic Aneurysm
- Aortic Arch Dissection: A Controversy of Classification
- Treatment Algorithms for Patients with (Sub)acute Type B Aortic Dissections
- Descending Thoracic Aortic Dissections
- Differences in Aortopathy in Patients with a Bicuspid Aortic Valve with or Without Aortic Coarctation
- Angina Chest pain
- Dyspnea Accompanied by Hypertension Crisis
- Sudden Coma or
- Shock of Unknown Etiology.

Another preferred embodiment of the lateral flow device according to the invention, further comprises a wicking pad for receiving and retaining samples after passing through the detection band.

In another preferred embodiment of the lateral flow device according to the invention the conjugate-binding protein is an FC-binding protein, preferably wherein the FC-binding protein is Protein G, Protein A or a Protein A/G fusion protein.

In another preferred embodiment of the lateral flow device according to the invention the conjugate-binding protein binds to the same biomarker as the protein antibody (to the AAD-protein) (in the "conjugate"). Then, the "conjugate-binding protein" can bind to the same epitope on the same biomarker as the "protein antibody" (in the "conjugate") or the "conjugate-binding protein" can bind to a different epitope on the same biomarker than the "protein antibody" (in the "conjugate").

In another preferred embodiment of the lateral flow device according to the invention the conjugate-binding protein binds to the same epitope on the same biomarker as the "protein antibody" (in the "conjugate").

In another preferred embodiment of the lateral flow device according to the invention the "conjugate-binding protein" can bind to a different epitope on the same biomarker than the epitope on the same biomarker that the "protein antibody" (in the "conjugate") binds to.

In another preferred embodiment of the lateral flow device according to the invention the detection agent conjugated to the antibody is selected from the group comprising metallic nanoparticles or nanoshells, non-metallic nanoparticles or nanoshells, enzymes, and fluorescent molecules.

In another preferred embodiment of the lateral flow device according to the invention the detection agent conjugated to the antibodies comprises nanoparticles or nanoshells of metallic gold.

In another preferred embodiment of the lateral flow device according to the invention the sample pad comprises a filter membrane for removing one or more components from the sample.

In another preferred embodiment of the lateral flow device according to the invention the one or more components removed from the sample by the filter membrane pad are cells, cellular material, fats, or particulate matter.

Another preferred embodiment of the lateral flow device according to the invention further comprises a control line located substantially perpendicular to the direction of flow of the sample along the strip. Preferably the conjugate pad further comprises an immunoglobulin conjugated to a detection agent so that in operation the sample flows from the sample pad to the conjugate pad and mobilizes the immunoglobulin conjugate which passes over the detection band without reactivity and crosses the control line, preferably wherein deposited at the control line is an antibody capable of binding to the mobilized immunoglobin conjugate as it crosses the control line, with optionally said binding at the control line being indicated by a visible color change.

In another preferred embodiment of the lateral flow device according to the invention as presented in the previous paragraph the immunoglobulin in the conjugate is from an animal species other than the mammal species from which the sample of bodily fluid is derived and/or the detection agent conjugated to the immunoglobulin is selected from the group comprising metallic nanoparticles or nanoshells, non-metallic nanoparticles or nanoshells, enzymes, and fluorescent molecules, preferably wherein the detection agent conjugated to the immunoglobins comprises nanoparticles or nanoshells of metallic gold.

In another preferred embodiment of the lateral flow device according to the invention the strip is formed of nitrocellulose.

In another preferred embodiment of the lateral flow device according to the invention the sample of bodily fluid is from a human, especially a patient.

In another preferred embodiment of the lateral flow device according to the invention the bodily fluid is selected from the group comprising whole blood, blood serum, and blood plasma.

Also included as a further aspect of the invention is a kit for detecting a protein released during an event leading to an acute aortic dissection (AAD-protein) in a sample of bodily fluid of a mammal, the kit comprising:
c) a lateral flow assay device as described above; and
d) an instruction information on how to use the lateral flow assay device as described above.

### Methods

Also included as a further aspect of the invention is a method for the detection of an AAD-protein in a body fluid of a mammal comprising using a lateral flow assay device as described above.

The present invention also includes a method of the detection of a first protein released during an event leading to an acute aortic dissection (1-AAD-protein) in a sample of bodily fluid of a mammal, the method comprising:
c) placing the sample onto a sample pad of a lateral flow assay device comprising:
   (1) a strip formed of a material enabling capillary flow of fluid along a portion of the strip;
   (2) the sample pad located proximal to one end of the strip for receiving the sample of the bodily fluid;
   (3) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   (4) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
   wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin; preferably is HMGI-C, with said antibody binding to the antigen of the amino acid sequence of SEQ ID NO: 1 or a fragment thereof;
d) interrogating the detection region for the presence of label particles to detect whether said AAD protein is present in the sample.

The present invention also includes a method for the immunoassay determination of acute aortic dissection (AAD) in a patient, said method comprising:
d) incubating a body fluid sample of the patient with
   a. at least one antibody to a first protein released during an event leading to an acute aortic dissection (1-AAD-protein) and
   b. a binding partner B for either 1-AAD-protein or the antibody
      wherein either the antibody or the binding partner B is conjugated to a detection agent,
      to form an immunological complex containing the detection agent;
e) isolating the immunological complex from the remaining sample; and
f) determining the detection agent either in the isolated complex or in the remaining sample as an indicator of the 1-AAD-protein in the sample;

thereby determining the occurrence of AAD in the patient,
wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin; preferably is HMGI-C, with said antibody binding to the antigen of the amino acid sequence of SEQ ID NO: 1 or a fragment thereof.

The invention is further described by way of Embodiments. It will be appreciated that the invention as claimed is not intended to be limited in any way by these Embodiments.

### EMBODIMENTS:

1. A lateral flow device for the detection of a first protein released during an event leading to an acute aortic dissection (1-AAD-protein) in a sample of bodily fluid of a mammal, the device comprising:
   a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising an conjugate binding site immobilized within the strip along a band located substantially perpendicular to the direction of flow of the sample along the strip so that when the mobilized 1-AAD-protein antibody conjugate in the sample contacts the conjugate binding site in the first detection band the presence of said 1-AAD-protein in the sample is indicated by the presence or absence of color,
   wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin,
   preferably wherein the presence or absence of color is a visible color change and the conjugate binding site is a first conjugate-binding protein
   OR - as an ALTERNATIVE -
   wherein the conjugate binding site is a surface-structure (site) of the biomarker, the 1-AAD-protein presented to the mobilized 1-AAD-protein antibody conjugate, to which the protein antibody could bind if not already bound.
2. The lateral flow device according to embodiment 1, wherein the 1- AAD-protein is aggrecan.
3. The lateral flow device according to embodiment 1, wherein the 1-AAD-protein is HMGI-C.
   3a. The lateral flow device according to embodiment 1, wherein the 1-AAD-protein is D-Dimer.
4. The lateral flow device according to embodiment 1, wherein said protein antibody binds to the antigen of the amino acid sequence of SEQ ID NO: 1 or a fragment thereof.
5. The lateral flow device according to embodiment 1, wherein the 1-AAD-protein is HMGI-C and the lateral flow device further comprises a second protein antibody binding to a second protein potentially also released during said event leading to an acute aortic dissection (2-AAD-protein), preferably wherein said protein antibody binding to said 1-AAD-protein binds to the antigen of the amino acid sequence of SEQ ID NO: 1 or a fragment thereof.
6. The lateral flow device according to embodiment 5, wherein the 2-AAD-protein is selected from a group of biomarkers for AAD including D-dimer smooth muscle actin myosin isozyme CK-MB and nucleic acid component (DISAMIN), and especially from aggrecan, smooth muscle myosin heavy chain, soluble aortic elastin or D-dimer, prefeably is D-Dimer, most preferably wherein the 1-AAD-protein is HMGI-C and the 2-AAD-protein is D-Dimer.
7. The lateral flow device according to any one of embodiments 5 or 6, wherein said second protein antibody to the 2-AAD-protein is conjugated to a second detection agent different from the one conjugated to said antibody to the 1-AAD-protein and said conjugate with the second protein antibody to the 2-AAD-protein is either located in said first conjugate pad or in a further conjugate pad.
8. The lateral flow device according to any one of embodiments 5 or 6, wherein said second protein antibody to the 2-AAD-protein is conjugated to the same detection agent as the one conjugated to said antibody to the 1-AAD-protein and said conjugate with the second protein antibody to the 2-AAD-protein is either located in said first conjugate pad or in a further conjugate pad.
9. The lateral flow device according to embodiment 8, wherein the first conjugate-binding protein in the first detection band will, when the second mobilized 2-AAD-protein antibody conjugate in the sample contacts the first conjugate-binding protein in the first detection band, the presence of the 2-AAD-protein in the sample is indicated by a visible color change.
10. The lateral flow device according to any one of embodiments 7 or 8, comprising a second detection band comprising a second conjugate-binding protein different from the first immobilized within the strip along a band located distanced from the first detection band substantially perpendicular to the direction of flow of the sample along the strip so that when the mobilized 2-AAD-protein antibody conjugate in the sample contacts the second conjugate-binding protein in the second detection band, the presence of the 2-AAD-protein in the sample is indicated by a visible color change.
11. The lateral flow device according to embodiment 1, wherein the lateral flow device further comprises one or more further protein antibody/ies binding to one or more further protein/s potentially also released during said event leading to an acute aortic dissection (X-AAD-protein/s).
12. The lateral flow device according to embodiment 11, wherein the 1-AAD-protein and the X-AAD-protein/s is/are selected from a group of biomarkers for AAD including D-dimer smooth muscle actin myosin isozyme CK-MB and nucleic acid component (DISAMIN), and especially from aggrecan, smooth muscle myosin heavy chain, soluble aortic elastin or D-dimer.
13. The lateral flow device according to embodiment 1, wherein the 1-AAD-protein is HMGI-C and the lateral flow device further comprises one or more further protein antibody/ies binding to one or more further protein/s potentially also released during said event leading to an acute aortic dissection (X-AAD-protein/s) preferably wherein said protein antibody binds to the antigen of the amino acid sequence of SEQ ID NO: 1 or a fragment thereof.
14. The lateral flow device according to embodiment 14, wherein the X-AAD-protein/s is/are selected from a group of biomarkers for AAD including D-dimer smooth muscle actin myosin isozyme CK-MB and nucleic acid component (DISAMIN), and especially from aggrecan, smooth muscle myosin heavy chain, soluble aortic elastin or D-dimer.
15. The lateral flow device according to any one of embodiments 11 to 14, wherein said one or more further protein antibody/ies to the X-AAD-protein/s is/are conjugated to the same detection agent as the one conjugated to said antibody to the 1-AAD-protein and said conjugate with the one or more further protein antibody/ies to the X-AAD-protein/s is/are located in said first conjugate pad and the first conjugate-binding protein in the first detection band will when the one or more further mobilized X-AAD-protein/s antibody/ies conjugate/s in the sample contact/s the first conjugate-binding protein in the first detection band, the presence of the one or more X-AAD-protein/s in the sample is/are indicated by a visible color change.
16. The lateral flow device according to any preceding embodiment, further comprising at least one MI-protein antibody binding to a protein indicative for acute myocardial infarction (Ml-protein) together with a separate MI-detection band comprising a further conjugate-binding protein different from the first and second immobilized conjugate-binding protein immobilized within the strip along a band located substantially perpendicular to the direction of flow of the sample along the strip so that when an at least one mobilized Ml-protein antibody sample conjugate in the sample contacts the further conjugate-binding protein in the MI-detection band the presence of said MI-protein in the sample is indicated by a visible color change.
17. The lateral flow device according to embodiment 16, wherein the MI-protein is selected from the group consisting of creatinine phosphokinase (CK) or its MB isozyme, cardiac myosin light chain, troponin T (TnT) and human heart fatty acids binding protein..
18. The lateral flow device according to any preceding embodiment, wherein the acute aortic dissection (AAD) is an acute aortic syndrome which comprises aortic dissection, including acute type A aortic dissection, and/or one or more of the following aortic dissections/acute aortic syndroms:
   - Penetrating Aortic Ulcer and Intramural Hematoma
   - Thoracic Aortic Aneurysm
   - Chronic Dissection-Indications with Treatment with Branched and Fenestrated Stent-grafts
   - Different Therapeutic Modalities for Aortic Arch Dissection Combined with Kommerell's Diverticulum
   - Secondary Interventions After Endovascular Repair of Aortic Dissections
   - False Aneurysms Complicating Internal Carotid Artery Dissection
   - Established Indications for the Invasive Treatment of a Thoracic Aortic Aneurysm
   - Aortic Arch Dissection: A Controversy of Classification
   - Treatment Algorithms for Patients with (Sub)acute Type B Aortic Dissections
   - Descending Thoracic Aortic Dissections
   - Differences in Aortopathy in Patients with a Bicuspid Aortic Valve with or Without Aortic Coarctation
   - Angina Chest pain
   - Dyspnea Accompanied by Hypertension Crisis
   - Sudden Coma or
   - Shock of Unknown Etiology.
19. The lateral flow device according to any one of the proceeding embodiments, further comprising a wicking pad for receiving and retaining sample after passing through the detection band.
20. The lateral flow device according to any one of the proceeding embodiments, wherein the conjugate-binding protein is an FC-binding protein.
21. The lateral flow device according to embodiment 20, wherein the FC-binding protein is Protein G, Protein A or a Protein A/G fusion protein.
22. The lateral flow device according to any one of the proceeding embodiments, wherein the detection agent conjugated to the antibody is selected from the group comprising metallic nanoparticles or nanoshells, non-metallic nanoparticles or nanoshells, enzymes, and fluorescent molecules.
23. The lateral flow device according to any one of the preceding embodiments, wherein the detection agent conjugated to the antibodies comprises nanoparticles or nanoshells of metallic gold.
24. The lateral flow device according to any one of the preceding embodiments, wherein the sample pad comprises a filter membrane for removing one or more components from the sample.
25. The lateral flow device according to any one of the preceding embodiments, wherein the one or more components removed from the sample by the filter membrane pad are cells, cellular material, fats, or particulate matter.
26. The lateral flow device according to any one of the preceding embodiments, further comprising a control line located substantially perpendicular to the direction of flow of the sample along the strip.
27. The lateral flow device according to embodiment 26, wherein the conjugate pad further comprises an immunoglobulin conjugated to a detection agent so that in operation the sample flows from the sample pad to the conjugate pad and mobilizes the immunoglobulin conjugate which passes over the detection band without reactivity and crosses the control line.
28. The lateral flow device according to embodiment 27, wherein deposited at the control line is an antibody capable of binding to the mobilized immunoglobin conjugate as it crosses the control line, with optionally said binding at the control line being indicated by a visible color change.
29. The lateral flow device according to embodiment 27 or embodiment 28, wherein the immunoglobulin in the conjugate is from an animal species other than the mammal species from which the sample of bodily fluid is derived.
30. The lateral flow device according to any one of embodiments 27 to 29, wherein the detection agent conjugated to the immunoglobulin is selected from the group comprising metallic nanoparticles or nanoshells, non-metallic nanoparticles or nanoshells, enzymes, and fluorescent molecules.
31. The lateral flow device according to embodiment 30, wherein the detection agent conjugated to the immunoglobins comprises nanoparticles or nanoshells of metallic gold.
32. The lateral flow device according to anyone of the preceding embodiments, wherein the strip is formed of nitrocellulose.
33. The lateral flow device according to any one of the preceding embodiments, wherein the sample of bodily fluid is from a human.
34. The lateral flow device according to any one of the preceding embodiments, wherein the bodily fluid is selected from the group comprising whole blood, blood serum, and blood plasma.
35. A method for the detection of an AAD-protein in a body fluid of a mammal comprising using a device of any one of embodiments 1 to 34.
36. A method of the detection of a first protein released during an event leading to an acute aortic dissection (1-AAD-protein) in a sample of bodily fluid of a mammal, the method comprising:
   e) placing the sample onto a sample pad of a lateral flow assay device comprising:
      (1) a strip formed of a material enabling capillary flow of fluid along a portion of the strip;
      (2) the sample pad located proximal to one end of the strip for receiving the sample of the bodily fluid;
      (3) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
      (4) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
      wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin; preferably is HMGI-C, with said antibody binding to the antigen of the amino acid sequence of SEQ ID NO: 1 or a fragment thereof;
   f) interrogating the detection region for the presence of label particles to detect whether said AAD protein is present in the sample.
37. A kit for detecting a protein released during an event leading to an acute aortic dissection (AAD-protein) in a sample of bodily fluid of a mammal, the kit comprising:
   e) a lateral flow assay device of any one of embodiments 1 to 34; and
   f) an instruction information on how to use the device of any one of embodiments 1 to 34.
38. A method for the immunoassay determination of acute aortic dissection (AAD) in a patient, said method comprising:
   g) incubating a body fluid sample of the patient with
      a. at least one antibody to a first protein released during an event leading to an acute aortic dissection (1-AAD-protein) and
      b. a binding partner B for either 1-AAD-protein or the antibody
         wherein either the antibody or the binding partner B is conjugated to a detection agent,
         to form an immunological complex containing the detection agent;
   h) isolating the immunological complex from the remaining sample; and
   i) determining the detection agent either in the isolated complex or in the remaining sample as an indicator of the 1-AAD-protein in the sample;
   thereby determining the occurrence of AAD in the patient,
   wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin; preferably is HMGI-C, with said antibody binding to the antigen of the amino acid sequence of SEQ ID NO: 1 or a fragment thereof.

The invention is further described with reference to the following examples. It will be appreciated that the invention as claimed is not intended to be limited in any way by these examples.

### Examples

### Example 1)

In Example 1 the antigen (biomarker) HMGI-C (High mobility group protein) was expressed, isolated and purified. For this, recombinant protein antigen expression tests in *E. coli* and mammalian cells were conducted.

### 1.a) Gene synthesis and sub-cloning in expression vector

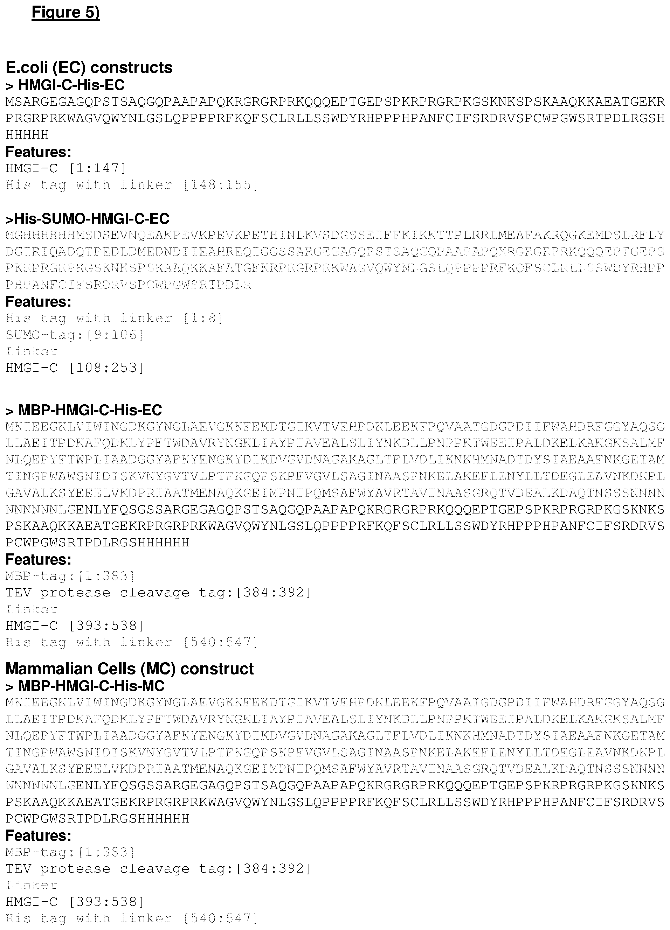

cDNA of HMGI with various tags (His, His-SUMO or MBP-His for bacterial expression and MBP-His for mammalian expression) were chemically synthesized with codon optimization for the corresponding expression host, then sub-cloned in an expression vector. The protein sequences are as follows and (partly) shown in Figure 5.

### 1.b) Protein sequences

**E.coli (EC) constructs**
   > **HMGI-C-His-EC** 
MSARGEGAGQPSTSAQGQPAAPAPQKRGRGRPRKQQQEPTGEPSPKRPRGRPKGSKNKSPSKAAQKKAEATGEKR
PRGRPRKWAGVQWYNLGSLQPPPPRFKQFSCLRLLSSWDYRHPPPHPANFCIFSRDRVSPCWPGWSRTPDLRGSH
HHHHH
**Features:**
   HMGI-C [1:147]
   His tag with linker [148:155]
**>His-SUMO-HMGI-C-EC** 
MGHHHHHHMSDSEVNQEAKPEVKPEVKPETHINLKVSDGSSEIFFKIKKTTPLRRLMEAFAKRQGKEMDSLRFLY
DGIRIQADQTPEDLDMEDNDIIEAHREQIGGSSARGEGAGQPSTSAQGQPAAPAPQKRGRGRPRKQQQEPTGEPS
PKRPRGRPKGSKNKSPSKAAQKKAEATGEKRPRGRPRKWAGVQWYNLGSLQPPPPRFKQFSCLRLLSSWDYRHPP
PHPANFCIFSRDRVSPCWPGWSRTPDLR
**Features:**
   His tag with linker [1:8]
   SUMO-tag:[9:106]
   Linker
   HMGI-C [108:253]
**> MBP-HMGI-C-His-EC** 
MKIEEGKLVIWINGDKGYNGLAEVGKKFEKDTGIKVTVEHPDKLEEKFPQVAATGDGPDIIFWAHDRFGGYAQSG
LLAEITPDKAFQDKLYPFTWDAVRYNGKLIAYPIAVEALSLIYNKDLLPNPPKTWEEIPALDKELKAKGKSALMF
NLQEPYFTWPLIAADGGYAFKYENGKYDIKDVGVDNAGAKAGLTFLVDLIKNKHMNADTDYSIAEAAFNKGETAM
TINGPWAWSNIDTSKVNYGVTVLPTFKGQPSKPFVGVLSAGINAASPNKELAKEFLENYLLTDEGLEAVNKDKPL
GAVALKSYEEELVKDPRIAATMENAQKGEIMPNIPQMSAFWYAVRTAVINAASGRQTVDEALKDAQTNSSSNNNN
NNNNNNLGENLYFQSGSSARGEGAGQPSTSAQGQPAAPAPQKRGRGRPRKQQQEPTGEPSPKRPRGRPKGSKNKS
PSKAAQKKAEATGEKRPRGRPRKWAGVQWYNLGSLQPPPPRFKQFSCLRLLSSWDYRHPPPHPANFCIFSRDRVS
PCWPGWSRTPDLRGSHHHHHH
**Features:**
   MBP-tag: [1:383]
   TEV protease cleavage tag:[384:392]
   Linker
   HMGI-C [393:538]
   His tag with linker [540:547]
**Mammalian Cells (MC) construct**
   > **MBP-HMGI-C-His-MC** 
MKIEEGKLVIWINGDKGYNGLAEVGKKFEKDTGIKVTVEHPDKLEEKFPQVAATGDGPDIIFWAHDRFGGYAQSG
LLAEITPDKAFQDKLYPFTWDAVRYNGKLIAYPIAVEALSLIYNKDLLPNPPKTWEEIPALDKELKAKGKSALMF
NLQEPYFTWPLIAADGGYAFKYENGKYDIKDVGVDNAGAKAGLTFLVDLIKNKHMNADTDYSIAEAAFNKGETAM
TINGPWAWSNIDTSKVNYGVTVLPTFKGQPSKPFVGVLSAGINAASPNKELAKEFLENYLLTDEGLEAVNKDKPL
GAVALKSYEEELVKDPRIAATMENAQKGEIMPNIPQMSAFWYAVRTAVINAASGRQTVDEALKDAQTNSSSNNNN
NNNNNNLGENLYFQSGSSARGEGAGQPSTSAQGQPAAPAPQKRGRGRPRKQQQEPTGEPSPKRPRGRPKGSKNKS
PSKAAQKKAEATGEKRPRGRPRKWAGVQWYNLGSLQPPPPRFKQFSCLRLLSSWDYRHPPPHPANFCIFSRDRVS
PCWPGWSRTPDLRGSHHHHHH
**Features:**
   MBP-tag: [1:383]
   TEV protease cleavage tag:[384:392]
   Linker
   HMGI-C [393:538]
   His tag with linker [540:547]

### 1.c) Expression & purification tests

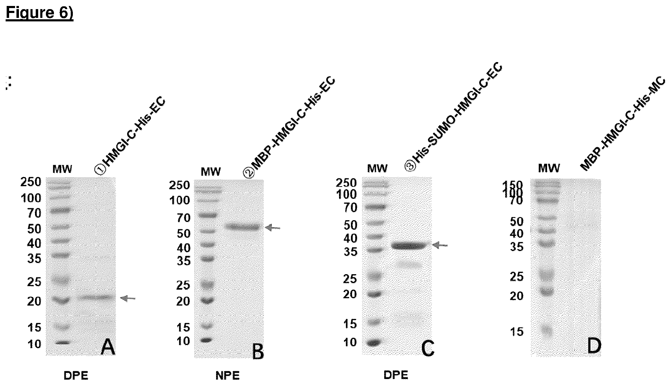

Proteins were expressed in *E. coli* or HEK cells, then purified by affinity against 6His-tag using Nickel resin and results were as follows:
- The His tagged and MBP tagged constructs were successfully expressed in E.coli (Figure 6,Gels A and B, respectively)
- The SUMO tag construct was successfully expressed in E.coli but with a lower purity than the other 2 constructs (Figure 6, Gel C)
- No expression was observed in Mammalian cells (Figure 6, Gel D)

Antigenic proteins obtained from *E. coli* were as follows:

| *Protein* | | *Concentration* | *Specification* | *Quantity* | *Yield* | *Purity* |
|---|---|---|---|---|---|---|
| ① | HMGI-C-His-EC | 0.45 mg/ml | 1.00 ml/vial | 2 vials | 0.90mg/1L | >90% |
| | DPE purification | | | | | |
| ② | MBP-HMGI-C-His-EC | 0.50 mg/ml | 1.20 ml/vial | 2 vials | 1.20mg/1L | >90% |
| | NPE purification | | | | | |
| ③ | His-SUMO-HMGI-C-EC | 0.85 mg/ml | 0.57 ml/vial | 2 vials | 0.97mg/1L | 80% |
| | DPE purification | | | | | |

### Example 2)

Antibodies binding to the antigen expressed, isolated and purified in Example 1 are produced.

### Publications:

1. Isselbacher EM. Disease of the aorta. Heart Disease, 6th edn. Braunwald E, Zipes DP, Libby P, Eds. W.B. Saunders Company, Philadelphia, 2001;1422-56.
2. Nienaber CA, Fattori R, Mehta RH, Richartz BM, Evangelista A, Petzsch M, et al. The International Registry of Acute Aortic Dissection (IRAD). Gender-Related differences in acute aortic dissection. Circulation. 2003;109:3014-21.
3. Pearson GD, Devereux R, Loeys B, Maslen C, Milewicz D, Pyeritz R, Ramirez F, et al. Report of the National Heart, Lung, and Blood Insti- tute and National Marfan Foundation Working Group on research in Marfan syndrome and related disorders. Circulation. 2008;118: 785-91.
4. Parapia LA, Jackson C. Ehlers-Danlos syndrome-a historical review. Br J Haematol. 2008;141 :32-5.
5. Callewaert B, Malfait F, Loeys B, De Paepe A. Ehlers-Danlos syn- dromes and Marfan syndrome. Best Pract Res Clin Rheumatol. 2008;22:165-89.
6. Roberts CS, Roberts WC. Dissection of the aorta with congenital malformation of the aortic valve. J Am Coll Cardiol. 1991;17: 712-6.
7. Mehta RH, Suzuki T, Hagan PG, et al. International Registry of Acute Aortic Dissection (IRAD). Investigators. Predicting death in patients with acute type a aortic dissection. Circulation. 2002;105: 200-6.
8. Daily PO, Trueblood HW, Stinson EB, Wuerflein RD, Shumway NE. Management of acute aortic dissections. Ann Thorac Surg. 1970;10: 237-47.
9. DeBakey ME, McCollum CH, Crawford ES, Morris GC, Howell J, Noon GP, Lawrie G. Dissection and dissecting aneurysms of the aorta: twenty-year follow-up of five hundred twenthy-seven patients treated surgically. Surgery. 1980;92:1118-34.
10. Anagnostopoulos CE, Prabhakar MJS, Kittle CF. Aortic dissections and dissecting aneurysms. Am J Cardiol. 1972;30:263-73.
11. Estrera AL, Huynh TT, Porat EE, Miller CC 3rd, Smith JJ, Safi HJ. Is acute type A aortic dissection a true surgical emergency? Semin Vasc Surg. 2002;15:75-82.
12. Miller DC. Acute dissection of the aorta: continuing need for earlier diagnosis and treatment. Mod Con Cardiovasc Dis. 1985;54:51-55.
13. Roberts WC. Aortic dissection: anatomy, consequences, and causes. Am Heart J. 1981;101 :195-214.
14. Aggrecan: a new biomarker for acute type A aortic dissection. Sci Rep 11, 10371 (2021). https://doi.org/10.1038/s41598-021-89653-y.
15. Upregulation of the high mobility group AT-hook 2 gene in acute aortic dissection is potentially associated with endothelial-mesenchymal transition. Histol Histopathol. 2011 Aug;26(8):1029-37. doi: 10.14670/HH-26.1029.

## Claims

1. A lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
wherein the 1-AAD-protein is selected from a group of biomarkers for acute aortic dissection (AAD) including D-dimer smooth muscle actin myosin isozyme CK-MB and nucleic acid component (DISAMIN), and especially HMGI-C, aggrecan, smooth muscle myosin heavy chain, or soluble aortic elastin.

2. The lateral flow device according to claim 1, wherein the 1-AAD-protein is aggrecan or wherein the 1-AAD-protein is HMGI-C or wherein the 1-AAD-protein is D-Dimer.

3. The lateral flow device according to any one of claims 1 or 2, wherein said protein antibody binds to the antigen of the amino acid sequence of SEQ ID NO: 1 or a fragment thereof.

4. The lateral flow device according to claim 1, wherein the 1-AAD-protein is HMGI-C and the lateral flow device further comprises one or more further protein antibody/ies binding to one or more further protein/s potentially also released during said event leading to an acute aortic dissection (X-AAD-protein/s) preferably wherein said protein antibody binding to said 1-AAD-protein binds to the antigen of the amino acid sequence of SEQ ID NO: 1 or a fragment thereof.

5. The lateral flow device according to claim 4, wherein the X-AAD-protein/s is/are selected from a group of biomarkers for AAD including D-dimer smooth muscle actin myosin isozyme CK-MB and nucleic acid component (DISAMIN), and especially from aggrecan, smooth muscle myosin heavy chain, soluble aortic elastin or D-dimer preferably is D-Dimer.

6. The lateral flow device according to any one of claims 4 or 5, wherein said one or more further protein antibody/ies to the X-AAD-protein/s is/are conjugated to the same detection agent as the one conjugated to said antibody to the 1-AAD-protein and said conjugate with the one or more further protein antibody/ies to the X-AAD-protein/s is/are located in said first conjugate pad and the first conjugate-binding protein in the first detection band will when the one or more further mobilized X-AAD-protein/s antibody/ies conjugate/s in the sample contact/s the first conjugate-binding protein in the first detection band, the presence of the one or more X-AAD-protein/s in the sample is/are indicated by a visible color change.

7. The lateral flow device according to any preceding claim, further comprising at least one MI-protein antibody binding to a protein indicative for acute myocardial infarction (Ml-protein) together with a separate MI-detection band comprising a further conjugate-binding protein different from the first and second immobilized conjugate-binding protein immobilized within the strip along a band located substantially perpendicular to the direction of flow of the sample along the strip so that when an at least one mobilized Ml-protein antibody sample conjugate in the sample contacts the further conjugate-binding protein in the MI-detection band the presence of said MI-protein in the sample is indicated by a visible color change; preferably wherein the MI-protein is selected from the group consisting of creatinine phosphokinase (CK) or its MB isozyme, cardiac myosin light chain, troponin T (TnT) and human heart fatty acids binding protein.

8. The lateral flow device according to any preceding claim, wherein the acute aortic dissection (AAD) is acute aortic syndrome which comprises aortic dissection, including acute type A aortic dissection, and/or one or more of the following aortic dissections/acute aortic syndroms:
- Penetrating Aortic Ulcer and Intramural Hematoma
- Thoracic Aortic Aneurysm
- Chronic Dissection-Indications with Treatment with Branched and Fenestrated Stent-grafts
- Different Therapeutic Modalities for Aortic Arch Dissection Combined with Kommerell's Diverticulum
- Secondary Interventions After Endovascular Repair of Aortic Dissections
- False Aneurysms Complicating Internal Carotid Artery Dissection
- Established Indications for the Invasive Treatment of a Thoracic Aortic Aneurysm
- Aortic Arch Dissection: A Controversy of Classification
- Treatment Algorithms for Patients with (Sub)acute Type B Aortic Dissections
- Descending Thoracic Aortic Dissections
- Differences in Aortopathy in Patients with a Bicuspid Aortic Valve with or Without Aortic Coarctation
- Angina Chest pain
- Dyspnea Accompanied by Hypertension Crisis
- Sudden Coma or
- Shock of Unknown Etiology.

9. The lateral flow device according to any one of the proceeding claims, wherein the conjugate-binding protein is an FC-binding protein, preferably wherein the FC-binding protein is Protein G, Protein A or a Protein A/G fusion protein.

10. The lateral flow device according to any one of the proceeding claims, wherein the detection agent conjugated to the antibody is selected from the group comprising metallic nanoparticles or nanoshells, non-metallic nanoparticles or nanoshells, enzymes, and fluorescent molecules, preferably wherein the detection agent conjugated to the antibodies comprises nanoparticles or nanoshells of metallic gold.

11. The lateral flow device according to any one of the preceding claims, further comprising a control line located substantially perpendicular to the direction of flow of the sample along the strip, preferably wherein the conjugate pad further comprises an immunoglobulin conjugated to a detection agent so that in operation the sample flows from the sample pad to the conjugate pad and mobilizes the immunoglobulin conjugate which passes over the detection band without reactivity and crosses the control line, preferably wherein deposited at the control line is an antibody capable of binding to the mobilized immunoglobin conjugate as it crosses the control line, with optionally said binding at the control line being indicated by a visible color change; even more preferably wherein the immunoglobulin in the conjugate is from an animal species other than the mammal species from which the sample of bodily fluid is derived.

12. A method for the detection of an AAD-protein in a body fluid of a mammal comprising using a device of any one of claims 1 to 11.

13. A method of the detection of a first protein released during an event leading to an acute aortic dissection (1-AAD-protein) in a sample of bodily fluid of a mammal, the method comprising:
a) placing the sample onto a sample pad of a lateral flow assay device comprising:
a. a strip formed of a material enabling capillary flow of fluid along a portion of the strip;
b. the sample pad located proximal to one end of the strip for receiving the sample of the bodily fluid;
c. a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
d. a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin; preferably is HMGI-C, with said antibody binding to the antigen of the amino acid sequence of SEQ ID NO: 1 or a fragment thereof;
b) interrogating the detection region for the presence of label particles to detect whether said AAD protein is present in the sample.

14. A kit for detecting a protein released during an event leading to an acute aortic dissection (AAD-protein) in a sample of bodily fluid of a mammal, the kit comprising:
a) a lateral flow assay device of any one of claims 1 to 11; and
b) an instruction information on how to use the device of any one of claims 1 to 11.

15. A method for the immunoassay determination of acute aortic dissection (AAD) in a patient, said method comprising:
a) incubating a body fluid sample of the patient with
a. at least one antibody to a first protein released during an event leading to an acute aortic dissection (1-AAD-protein) and
b. a binding partner B for either 1-AAD-protein or the antibody
wherein either the antibody or the binding partner B is conjugated to a detection agent,
to form an immunological complex containing the detection agent;
b) isolating the immunological complex from the remaining sample; and
c) determining the detection agent either in the isolated complex or in the remaining sample as an indicator of the 1-AAD-protein in the sample;
thereby determining the occurrence of AAD in the patient,
wherein the 1-AAD-protein is selected from a group of biomarkers for AAD including D-dimer smooth muscle actin myosin isozyme CK-MB and nucleic acid component (DISAMIN), and especially HMGI-C, aggrecan, smooth muscle myosin heavy chain, or soluble aortic elastin; preferably is HMGI-C, with said antibody binding to the antigen of the amino acid sequence of SEQ ID NO: 1 or a fragment thereof.
